# EUROPEAN PATENT SPECIFICATION

(11) **EP 2 448 562 B1**
(45) Date of publication and mention of the grant of the patent: **06.11.2013**
(21) Application number: 10730726.6
(22) Date of filing: 01.07.2010
(51) Int. Cl.: A61K 9/20, A61K 31/4535

(54) **RALOXIFENE COMPOSITION**
RALOXIFEN-ZUSAMMENSETZUNG
COMPOSITION DE RALOXIFÈNE

(30) Priority: 02.07.2009 US 222915 P
(43) Date of publication of application: 09.05.2012
(73) Proprietor: Synthon B.V., 6545 CM Nijmegen (NL)
(72) Inventor: ABUKHALIL, Asad, NL-6545 CM Nijmegen (NL)
(74) Representative: van den Broek, Ludovicus A.G.M.
(86) International application number: PCT/EP2010/004125
(87) International publication number: WO 2011/000581

(56) References cited:
- EP-A1- 0 670 162
- WO-A1-97/35571
- WO-A1-2009/049643
- WO-A2-2006/052254
- US-A1- 2002 031 548
- US-A1- 2005 282 784
- US-A1- 2009 162 444
- US-A1- 2010 003 319
- FREED A L ET AL: "pH control of nucleophilic/electrophilic oxidation", INTERNATIONAL JOURNAL OF PHARMACEUTICS, ELSEVIER BV, NL, vol. 357, no. 1-2, 5 June 2008 (2008-06-05), pages 180-188, XP022637095, ISSN: 0378-5173, DOI: DOI:10.1016/J.IJPHARM.2008.01.061 [retrieved on 2008-03-10]

## Description

### BACKGROUND OF THE INVENTION

Raloxifene, or (6-hydroxy-2-(4-hydroxy-phenyl)benzo[b]thien-3-yl)-(4-(2-(1-piperidinyl)ethoxy)phenyl) methanone of the formula 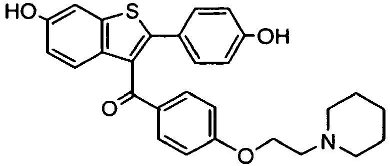 is a pharmaceutically active compound, indicated for treatment and/or prevention of osteoporosis in women. In pharmaceutical formulations, it is used in the form of a hydrochloride salt. Pharmaceutical formulations comprising raloxifene hydrochloride are marketed, e.g., under the brand name Evista® by Eli Lilly.

The marketed formulation is a film-coated tablet comprising 60 mg of raloxifene hydrochloride. The excipients in the composition include povidone (polyvinylpyrrolidone), polysorbate 80, anhydrous lactose, lactose monohydrate, crospovidone, magnesium stearate, hypromellose, macrogol 400 (polyethylene glycol), carnauba wax, modified pharmaceutical glaze, and propylene glycol.

The raloxifene compound as well as the raloxifene hydrochloride compound have been disclosed, e.g., in US 4418068. Additional synthetic schemes as well as crystalline raloxifene hydrochloride have been disclosed in WO 96/09045 (US 5731327). The use of raloxifene in treating bone loss and compositions therefor have been described in US 5393763 and US 5478847. Raloxifene hydrochloride is very slightly soluble in each of water (627µg/ml), gastric fluid, and intestinal fluid. Due in part to the low water solubility, the bioavailability of raloxifene hydrochloride is generally low. Accordingly, several approaches have been proposed to increase the bioavailability of raloxifene hydrochloride in pharmaceutical formulations.

EP 670162 B1 (US 5811120, US 5972383) disclosed an orally administered pharmaceutical formulation comprising raloxifene or a pharmaceutically acceptable salt thereof (preferably hydrochloride) in combination with (i) a surfactant, (ii) a water-soluble diluent, and (iii) optionally a hydrophilic binder such as polyvinylpyrrolidone. According to the patentees the surfactant and water-soluble diluent enhance the bioavailability of raloxifene hydrochloride. The surfactant is preferably a polyoxyethylene sorbitan fatty acid ester such as polysorbate 80 and the water-soluble diluent is preferably a sugar, especially lactose. While disclosed as "optional," the hydrophilic binder is nonetheless preferably present, and most preferably is polyvinylpyrrolidone. Correspondingly, every claim of EP 670162 B1, US 5811120, and US 5972383 requires the presence of polyvinylpyrrolidone in the pharmaceutical formulation.

Subsequently WO 97/35571 (US 6458811, US 6797719, and US 6894064) disclosed micronizing raloxifene hydrochloride in order to increase bioavailability. The particulate raloxifene hydrochloride has a median particle size of 25 microns or less, preferably 5 to 20 microns. The particulates generally have a size distribution such that at least 90% have a particle size of less than 50 microns, preferably less than 35 microns. The claims in US 6797719 and US 6894064 indicate that the use of a surfactant and water-soluble diluent (as in the earlier EP 670162 B1 composition discussed above) are preferably used in combination with the micronized raloxifene hydrochloride.

Alternatively, EP 826682 B1 disclosed amorphous raloxifene hydrochloride as having improved solubility and bioavailability. The amorphous form is generally stabilized to prevent conversion to a crystalline form via the use of povidone, PEG, or other known stabilizer materials.

WO 2006/052254 A2 disclosed a compressed pharmaceutical formulation comprising raloxifene hydrochloride and starch in an amount of greater than about 25%, preferably 45 to 75%. The use of such large amounts of starch is purported to allow the use of non-micronized raloxifene hydrochloride (i.e., large particles) while still achieving satisfactory dissolution rates and bioavailability. The tablets are preferably made via a wet double compression process. The examples compare a lactose/microcrystalline cellulose-based composition and a low starch-content composition to several high starch-content compositions (60-75% starch). The high starch-content compositions, which did not contain a water-soluble diluent such as lactose, had the best dissolution.

WO 2009/049643 A1 disclosed another raloxifene composition. A super disintegrant such as sodium starch glycolate (Primojel) is purported to enhance bioavailability and to increase drug release. The composition is typically prepared by wet granulation and generally contains a surfactant such as Poloxamer 407. A pH adjusting agent such as citric acid can be added to the composition and a pH of 6.0 is mentioned. The use of citric acid monohydrate in an amount of 2% is shown in two of the examples. The other excipients such as binder, diluent, etc., are not described as being specifically limited, but the examples show the use of dibasic calcium phosphate (a water-insoluble diluent) and microcrystalline cellulose. The size of the raloxifene hydrochloride particles is not described or addressed.

US 2005/0249814 disclosed pharmaceutical compositions having improved solubility that comprise a hydrophobic drug and a compound having at least one carboxylic acid moiety in a ratio of 1:0.1 to 25, respectively. The disclosure is primarily directed to Ziprasidone HCl, but several drugs including raloxifene are also mentioned as being suitable. The compound having the carboxylic acid moiety includes a variety of acids, the most preferred being citric acid. The examples show that the amount of acid needed to be significantly effective (at least in the case of Ziprasidone) is quite large as a percentage of the capsule composition; e.g., 36% and 53% citric acid in Examples 5 and 6.

US 2002/0031548 disclosed several excipient combinations for raloxifene HCl; i.e., examples 7-9. These recipes include lactose, microcrystalline cellulose, starch, and a surfactant. Also included is ascorbic acid or sodium ascorbate as an antioxidant in amounts of 1.5 to 10%. The relative amount of raloxifene is not provided, however, and thus the percentages recited do not correspond to the percentages of tablet weight. No dissolution or bioavailability data is provided regarding these raloxifene formulations and no discussion of particle size or dissolution issues associated therewith is set forth.

While various approaches for providing raloxifene with suitable dissolution and/or bioavailability have been put forth, it would be desirable to provide a formulation based on a water-soluble diluent that does not require micronized raloxifene.

### SUMMARY OF THE INVENTION

The present invention relates to raloxifene tablets and processes for making the same. Accordingly a first aspect of the invention relates to a pharmaceutical tablet, comprising: (a) an effective amount of non-micronized raloxifene, or a pharmaceutically acceptable salt thereof; (b) at least 25% by weight of lactose; (c) 5 to 25% by weight of microcrystalline cellulose; optionally (d) a binder; and optionally (e) a disintegrant. The combination of microcrystalline cellulose with lactose can surprisingly provide improved release for non-micronized raloxifene (defined hereinafter). In a preferred embodiment, the tablet exhibits a dissolution profile such that at least 85% and preferably 90% of the raloxifene is released in 30 minutes and preferably at least 95% raloxifene release in 60 minutes under suitable dissolution conditions, such as USP apparatus Type II, in 1 liter aqueous solution containing 0.1 % polysorbate 80 at paddle speed of 50 rpm. Such dissolution can be achieved without the inclusion of a surfactant into the tablet, though the use thereof is not excluded. In an advantageous embodiment, the tablet further contains an organic acid, such as citric acid, or a salt thereof which can improve the total release of raloxifene.

Described herein is also a raloxifene tablet comprising (a) an effective amount of raloxifene, or a pharmaceutically acceptable salt thereof; and (b) 0.1 to 1.0% by weight of citric acid or a salt thereof. The presence of small amounts of citric acid can improve the *in vitro* dissolution of raloxifene, whether micronized or non-micronized.

Another aspect of the invention relates to a process, which comprises:
(1) mixing non-micronized raloxifene, or a pharmaceutically acceptable salt thereof, having a median particle size of at least 25 microns and/or a d₉₀ of at least 50 microns; microcrystalline cellulose; a disintegrant; and optionally a binder, preferably polyvinylpyrrolidone, to form a blend;
(2) wet granulating said blend with water to form granules;
(3) mixing said granules with at least lactose to form a tablet blend; and
(4) tabletting said tablet blend by compression to form at least one tablet;
wherein lactose comprises at least 25% by weight of said tablet and said microcrystalline cellulose comprises 5 to 25% by weight of said tablet.

A further aspect of the invention relates to a process for making a tablet without the aid of water, which comprises:
(1) blending in one or more steps at least non-micronized raloxifene, or a pharmaceutically acceptable salt thereof, having a median particle size of at least 25 microns and/or a d₉₀ of at least 50 microns; microcrystalline cellulose; lactose; a binder; and a disintegrant to form a tablet blend; and
(2) tabletting said tablet blend by compression to form at least one tablet;
wherein lactose comprises at least 25% by weight of said tablet and said microcrystalline cellulose comprises 5 to 25% by weight of said tablet.

### BRIEF DESCRIPTION OF THE DRAWINGS

Figure 1 represents the dissolution curves for each of the tablets according to Example 1.

Figure 2 represents the dissolution curves for each of the tablets according to Example 2.

Figure 3 represents the dissolution curves for each of the tablets according to Example 3.

Figure 4 represents the dissolution curves for each of the tablets according to Example 4.

Figure 5 represents the dissolution curves for each of the tablets according to Example 5.

### DETAILED DESCRIPTION OF THE INVENTION

The present invention relates to the discovery of a simple, convenient excipient system for achieving good *in vitro* dissolution release of non-micronized raloxifene. In one embodiment the system is based on a water-soluble diluent, lactose, in combination with microcrystalline cellulose (hereinafter frequently "MCC"). In another, the raloxifene tablet contains an organic acid or salt thereof, which can be used alone or in combination with the previous embodiment.

The tablets of the invention contain an effective amount of raloxifene or a pharmaceutically acceptable salt thereof (for convenience the base and salt are referred to collectively as "raloxifene" hereinafter unless otherwise indicated). Typically the raloxifene is raloxifene hydrochloride. An effective amount of raloxifene is known in the art and is generally within the range of 10 to 200 mg, more typically 20 to 100 mg, such as 30 or 60 mg (amounts of raloxifene refer to the amount of base whether in base or salt form). In terms of percentage, generally the amount of raloxifene is 10 to 30%, more typically 15 to 25% of the tablet. The raloxifene can be crystalline or amorphous, and is typically crystalline (unless otherwise stated in the description all percentages are by weight).

The raloxifene used in the tablets of the invention is non-micronized. As used herein, "non-micronized" raloxifene means that the particles of the raloxifene used to make the tablets had a median particle size of at least 25 microns (i.e., d₅₀ is at least 25 microns) and/or a d₉₀ of at least 50 microns. The "particle size" refers to the equivalent spherical diameter of the particles as determined by laser light diffraction scattering. Note that the term "non-micronized" does not exclude milling of the raloxifene. Instead, the term is merely a characterization of the size of the raloxifene used to make the tablets and is thus in distinction to the smaller, "micronized" raloxifene taught in US 6,458,811. Also, the raloxifene size is determined pretabletting. While it is thought that the raloxifene remains the same particle size/distribution, or possibly becomes larger, during tabletting, measuring the raloxifene particle size post-tabletting is cumbersome and not always precise. Accordingly, a tablet made from non-micronized raloxifene is a tablet containing non-micronized raloxifene for purposes of the present invention.

The particle size of raloxifene, i.e., the equivalent spherical diameter, is determined by laser light diffraction scattering. Devices for determining the equivalent spherical diameter are commercially available and the technique is well known in the art; e.g., US 6,458,811. The non-micronized raloxifene typically has a d₅₀ of at least 25 microns, such as 26-40 microns, and a d₉₀ of at least 50 microns, such as 51 to 75 microns.

Turning to the first embodiment of the invention, the water-soluble diluent used in the tablet is lactose. The lactose can be any pharmaceutical form including anhydrous, monohydrate, etc. The amount of the water-soluble diluent in the tablet of the invention is at least 25% of the tablet weight, typically at least 30%, more typically at least 40%. Usually the water-soluble diluent comprises 35-70%, more typically 40-60% and in some embodiments 45-55% of the tablet weight. Generally, though not necessarily, the water-soluble diluent is the largest portion of the tablet.

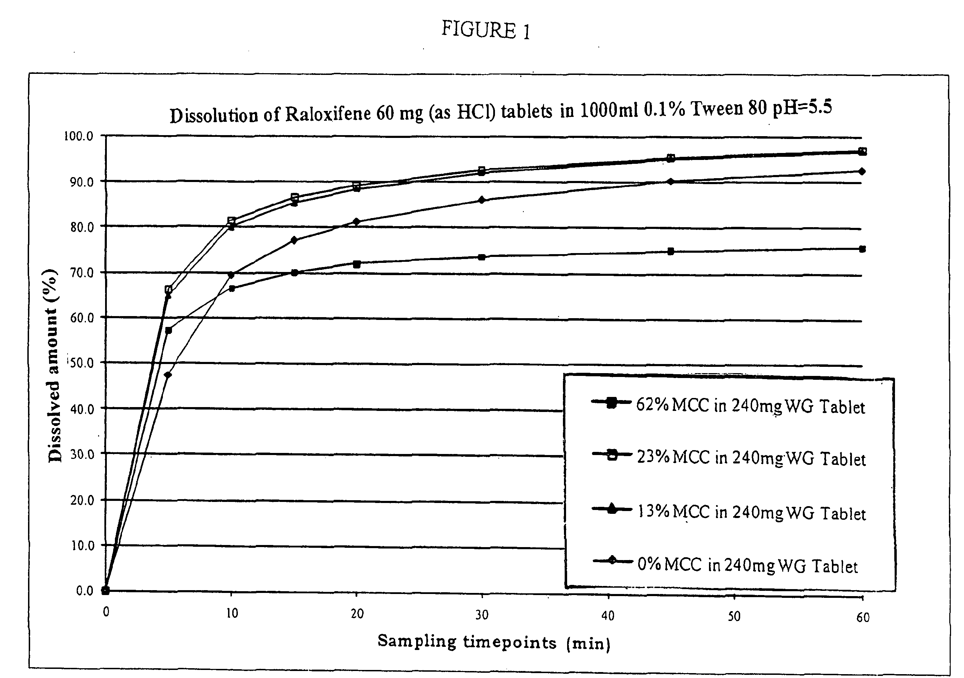

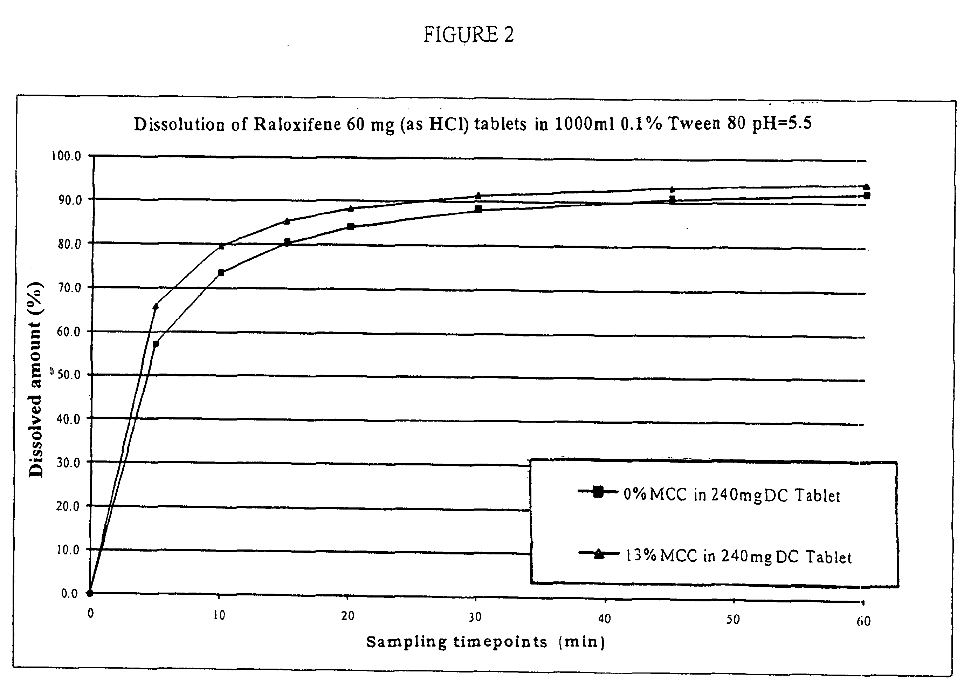

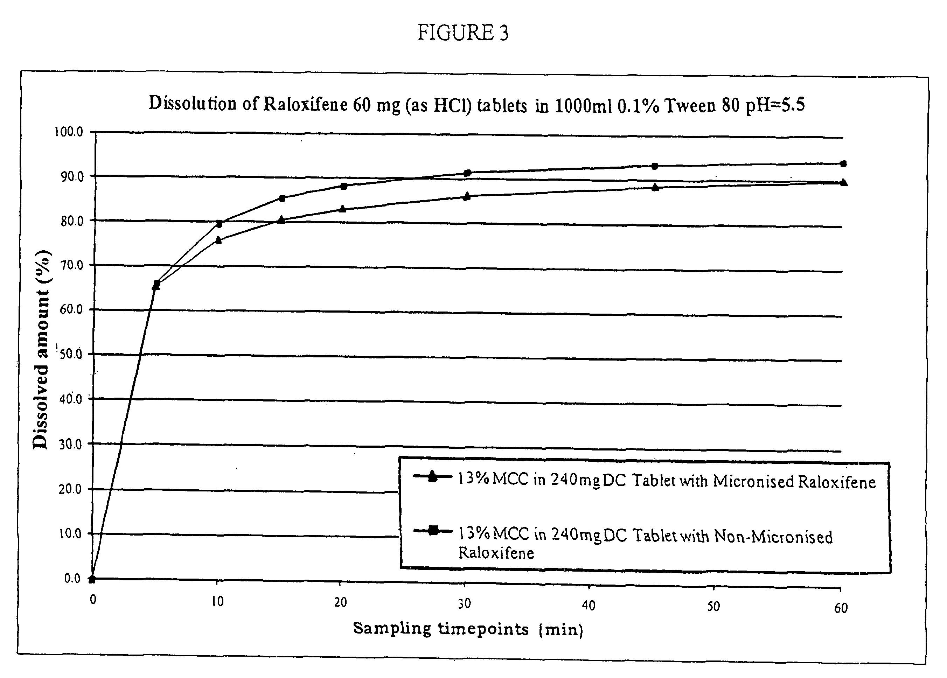

The MCC used in the tablet of the invention comprises up to 25% of the tablet weight. It is believed that restricted amounts of MCC in combination with a greater amount of lactose provides for superior dissolution of non-micronized raloxifene. The combination of MCC and water-soluble diluent lactose can be synergistic. That is, the use of the two fillers can provide improved release of non-micronized raloxifene in comparison to the use of each filler alone. Example 1, set forth below, compares tablets made by wet granulation having 63% lactose, 63% MCC, or mixtures, with the dissolution results represented in Fig. 1. While the "all lactose" tablet had superior dissolution to the "all MCC" tablet, replacing some of the lactose with the inferior MCC surprisingly improved the dissolution beyond the "all lactose" tablet. That is, using 13% MCC and 50% lactose gave superior release over the 63% lactose tablet. Increasing the MCC content while maintaining the tablet weight (23% MCC and 40% lactose) had little improvement over the 13% MCC tablet. Example 2 and Fig. 2 show that a similar improvement is obtained in a direct compression tablets; e.g., the lactose-only tablet does not release as quickly or as much as the lactose/MCC tablet). Also surprisingly, the improvement in dissolution brought out by the combination of MCC and lactose is not seen (or not as dramatic) for micronized raloxifene. Example 3 and Fig. 3 show that micronized raloxifene exhibits slower release than non-micronized raloxifene in the MCC/lactose blend of the invention. That larger particles release faster is in contrast to the teachings of US 6,458,811.

It appears that the amount of MCC should be limited in order to obtain the beneficial increase in dissolution. Generally the amount of MCC is within the range of 5 to 25%, more typically 10 to 20 %, including 10 to 15%, based on the total weight of the tablet. Amounts greater than 25% may provide little or no further advantage and thus are generally not desired. Moreover, such higher amounts may actually reduce the dissolution of the tablet. Also, the amount of water-soluble diluent appears to be important. The ratio of MCC to water-soluble diluent (lactose) is generally 1 part MCC:1-12 parts of water-soluble diluent; typically 1:1.5-10; and preferably about 1:2-5, including about 1:4 (MCC to water-soluble diluent, respectively, in each instance). While not wishing to be bound by theory, it is speculated that the combination of water-soluble diluent and MCC serves to prevent agglomeration of the raloxifene particles during the tabletting process. By decreasing the amount of agglomeration of raloxifene particles, the dissolution is improved. The "microcrystalline cellulose" is a well known pharmaceutical excipient. In the compositions of the present invention, its quality preferably corresponds to the specification of the Ph.Eur. In an advantageous mode, the MCC of a specific particle size may be used. The medium particle size (D50) of MCC may be between 10µm and 200µm, preferably between 20µm and 180µm, more preferably between 20µm and 150µm and most preferably between 30µm and 75µm.

The filler combination of water-soluble diluent and MCC typically comprises the majority of the tablet and often falls within the range of 50 to 80% of the tablet weight. The tablet generally weighs three to eight times the amount of raloxifene. Larger tablet weights can improve dissolution, likely because the chance for agglomeration is reduced; e.g., more excipient between raloxifene particles. But larger tablet weights can be more inconvenient in terms of administration and have increased cost. For a 60 mg dose of raloxifene (calculated as free base), a tablet of the invention normally has a tablet weight between 200 and 400 mg. The current commercially sold tablet has a tablet weight of about 240 mg. Accordingly, it is desirable that the tablet of the invention have a similar weight, i.e., 220 to 250 mg, or more, e.g., 260 to 200µm, preferably between 20µm and 180µm, more preferably between 20µm and 150µm and most preferably between 30µm and 75µm.

The filler combination of water-soluble diluent and MCC typically comprises the majority of the tablet and often falls within the range of 50 to 80% of the tablet weight. The tablet generally weighs three to eight times the amount of raloxifene. Larger tablet weights can improve dissolution, likely because the chance for agglomeration is reduced; e.g., more excipient between raloxifene particles. But larger tablet weights can be more inconvenient in terms of administration and have increased cost. For a 60 mg dose of raloxifene (calculated as free base), a tablet of the invention normally has a tablet weight between 200 and 400 mg. The current commercially sold tablet has a tablet weight of about 240 mg. Accordingly, it is desirable that the tablet of the invention have a similar weight, i.e., 220 to 250 mg, or more, e.g., 260 to 360mg. Specifically contemplated higher tablet weights include 260, 280, 300, 320, 340, and 350 mg as well as all the tablet weights between such weights. For clarity, all references herein to tablet weight, including references to percentages based on total tablet weight, refer to the uncoated tablet and do not include the weight/mass of any optional coating(s) on the tablet.

The tablet may optionally further contain a binder. The binder, when present, is usually contained in minor amounts such as 1 to 10%, though higher amounts could be used. The binder is preferably a hydrophilic binder such as polyvinylpyrrolidone; cellulose derivatives (e.g., hydroxypropyl cellulose); or polyethylene glycol. Preferably the binder is polyvinylpyrrolidone which, in addition to providing tablet binding properties, generally enhances the dissolution of raloxifene.

The tablet may optionally contain a disintegrant. To enhance dissolution as well as tablet disintegration, the tablet of the invention normally contains a disintegrant in an amount of 1 to 10%. Typical disintegrants include cross-linked polyvinylpyrrolidone, cross-linked sodium carboxymethylcellulose, starches, sodium starch glycolate, clays, and celluloses. A preferred disintegrant is cross-linked polyvinylpyrrolidone, also known as crospovidone.

The tablet of the invention may also contain an organic acid or salt thereof. The organic acid can be citric acid, ascorbic acid, etc. The presence of the organic acid can improve the rate of release of raloxifene and/or the total amount of raloxifene released. In general the amount of the organic acid is within the range of 0.1 to 10%, more typically 0.1 to 5%. In a particular aspect of the invention, the use of small amounts of acid, especially citric acid, provide for surprisingly large effects. Thus, a tablet containing 0.1 to 2.0%, more preferably 0.1 to 1.0% including 0.1 to 0.5%, of organic acid, especially of citric acid, is a preferred embodiment of the invention.

Indeed, a second embodiment of the invention relates to the use of citric acid, or a salt thereof, to improve the dissolution rate and/or the total amount of raloxifene released. This effect is independent of the MCC/water-soluble diluent filler combination described above. The amount of citric acid, or its salt, needed to have a beneficial effect is surprisingly small and typically is in the range of 0.1 to 1.0%, often 0.1 to 0.5%. Accordingly another embodiment of the invention is a raloxifene tablet containing an effective amount of micronized or non-micronized raloxifene or a pharmaceutically effective salt thereof; and 0.1 to 1.0% of citric acid or a salt thereof. The tablet often contains a water-soluble diluent, typically in amounts of at least 25% and often 30-70%. The other binders and disintegrants, as described above, can also be used in this embodiment in the same amounts as previously set forth.

All embodiments of the tablets of the invention can contain other conventional excipients. For example, lubricants such as magnesium stearate; glidants or flow agents such as colloidal silicon dioxide; coloring agents; flavorants; etc.; as are known in the art. A surfactant may also be included to assist in dissolution performance as described in US 5,811,120. Specifically a surfactant such as polysorbate 80, can be incorporated into the tablet. In certain preferred embodiments, however, it is desired to reduce or avoid entirely the inclusion of a surfactant. Thus a surfactant may be limited to less than 0.5%, preferably less than 0.2%, and more preferably 0% of the tablet weight. In these embodiments, the use of the combined water-soluble diluent and MCC optionally with polyvinylpyrrolidone and/or citric acid, can provide the desired dissolution without the need to resort to a surfactant. Similarly, though starch can be used in the tablets of the invention, it is preferred in some embodiments that the amount of starch is limited or eliminated; e.g., less than 10%, preferably less than 2%, more preferably 0% of starch in the tablet. Likewise, a crosslinked starch such as sodium starch glycolate is preferably limited to less than 10%, more preferably less than 5%, still more preferably less than 2%, and most preferably 0% of the tablet.

The tablets of the invention are generally immediate release tablets, meaning that at least 80% of the raloxifene is released within 30 minutes from the start of an *in vitro* dissolution test. Preferably the dissolution is quicker and substantially complete within 60 minutes. Accordingly, the dissolution profile preferably exhibits at least 85% release, more preferably at least 90% release of raloxifene in 30 minutes. The dissolution should achieve at least 85% release, preferably at least 90% release, and more preferably at least 95% release of raloxifene in 60 minutes. Similarly regarding *in vivo* performance, the tablets of the invention preferably are bioequivalent to the current commercially sold immediate release tablet, e.g., Evista®. That is, the preferred tablets can receive an "AB" rating from the U.S. FDA and/or are otherwise generically substitutable with Evista®.

Dissolution testing is well known in the art. A suitable dissolution method is normally required by drug regulatory authorities such as the U.S. FDA. A "suitable" dissolution method is one that reasonably predicts or confirms the adequacy of the tablet to perform in vivo and can discriminate an improperly made or underperforming tablet from a correct tablet that will be safe and effective. Workers skilled in the art commonly select the appropriate conditions including the apparatus, dissolution container, dissolution media, the volume of the media, the paddle speed (e.g., 50 rpm vs. 75 rpm), etc., in order to create a suitable dissolution method for a given tablet. For purposes of the present invention a "suitable" dissolution method is one that a regulatory authority has approved/not objected to for a given tablet. For purposes of the present invention, the dissolution profile measurements mentioned above are satisfied if the tablets meet such measurements in either a suitable dissolution test or in a dissolution test run under the following conditions: USP apparatus Type II, in 1 liter aqueous solution containing 0.1% polysorbate 80 (initial pH of 5.5) at a paddle speed of 50 rpm and a temperature of 37.5°C.

The tablets of the present invention can be coated if desired. Such coating is typically for aesthetic, handling, and/or stability reasons. These types of coatings are commercially available and well known in the art. Preferably an uncoated tablet of the invention disintegrates in water in less than 7 minutes, preferably less than 5 minutes, and in some embodiments less than 4 minutes. A coated tablet typically disintegrates in water in less than 15 minutes and generally within 6 to 10 minutes.

The tablets of the invention can be made by any convenient or suitable tabletting techniques known in the art. In general, tablets can be made by wet methods or dry methods. Wet methods refer to the technique known as wet granulation wherein a granulating fluid is used to form granules of the active (here raloxifene) with some or all of the excipients. Dry methods include direct compression as well as dry granulation also known as slugging. A tablet made by such dry methods (hereinafter a "dry-made tablet") is generally distinguishable from a wet made tablet under microscopic examination. Also, a dry-made tablet may have different physical characteristics than a corresponding wet granulation made tablet. It is another aspect of the present invention that the tablets of the invention can be dry-made and preferably exhibit the preferred dissolution profiles described-above. For clarity, a dry-made tablet may be subsequently coated by a process that involves a liquid but does not lose its dry-made status for purposes of the present invention.

In forming the tablets using wet granulation, generally water is used as the granulating fluid, though organic solvents may be used if desired. The conditions of granulation are preferably selected so as to encourage/attain smaller granules rather than larger granules as smaller granules generally provide for faster and/or more complete dissolution. Such conditions include the volume of liquid, the rate of addition, the impeller and/or chopper speed, and the duration of the granulating. The granulating fluid preferably contains water and optionally a surfactant, a hydrophilic binder, and/or an organic acid. Water-soluble diluent could in theory be incorporated, at least partly, into the granulating liquid, e.g., dissolved therein. But it is normally desired to not include any diluent or filler in the granulating liquid. Indeed, it is generally preferred that if a binder is present, such as polyvinylpyrrolidone, such binder is not dissolved or suspended in the granulating fluid. The granulating fluid is added to a blend containing non-micronized raloxifene. The blend typically contains a portion of the filler and the optional binder. Frequently the blend comprises the non-micronized raloxifene, MCC, and optionally polyvinylpyrrolidone and/or a disintegrant. The granulating fluid is combined under granulating conditions to form granules which may be dried, sieved, and/or milled. The resulting granules are blended with additional excipients to form a tablet blend; i.e., a blend suitable for compressing into a tablet. The additional excipients include additional filler and optionally binder and/or disintegrant. Additionally a lubricant and/or glidant are often included in the tablet blend. The tablet blend can be formed in one or more blending steps; e.g., filler, lubricant, and disintegrant are pre-blended and then added to the granules, or, adding sequentially the filler, lubricant, and disintegrant to the granules, etc., wherein each step can be performed one or more times. Often the water-soluble diluent is provided entirely in the tablet blend, e.g., as extragranular excipient. Likewise the entirety of the MCC is often provided in the granulate, e.g., intra-granular excipient. Nonetheless, such separation is not required in making the tablet of the present invention. Once the tablet blend is formed, tablets can be made by compression as is known in the art. The size and shape of the tablets is not particularly limited.

Alternatively the tablets of the invention can be dry-made. Direct compression is the simplest technique. In general the non-micronized raloxifene is blended in one or more steps (each conducted one or more times) with the excipients until a tablet blend is formed. This tablet blend is then compressed to form tablets. In a slightly more complicated process, the non-micronized raloxifene is dry granulated with filler and optionally binder to form dry granules or slugs. The slugs are then blended with the remaining excipients to form a tablet blend. The water-soluble diluent tablets of the present invention generally do not require a binder to accommodate dry-made methods. Nonetheless, a hydrophilic binder such as polyvinylpyrrolidone can be advantageously incorporated to assist in the dissolution profile.

The tablets of the invention can be used to treat any raloxifene-treatable disease or condition, such as bone loss, breast cancer, etc.

The invention will be further described with reference to the following nonlimiting examples.

### Examples

The dissolution test used in each of the following examples comprises USP apparatus Type II, in 1 liter aqueous solution containing 0.1% polysorbate 80 (initial pH of 5.5) at a paddle speed of 50 rpm and a temperature of 37.5°C. Generally an average of 3 to 6 tablets is taken as the tablet value.

### Example 1

Tablets according to the below compositions were made via wet granulation. Specifically, Raloxifene was mixed with MCC, polyvinylpyrrolidone (PVP) and Crospovidone. The binder solution of water and citric acid was then added to the powder and mixed accordingly. The wet granules were dried and lactose and magnesium stearate were blended with the dried granules in respective manner. The blend was then compressed to form tablets.

**Table A - Tablets with 0% MCC made with wet granulation**

| **Ingredient** | **Function** | **Ratio (%)** | **Unit Ratio (mg)** |
|---|---|---|---|
| **Intragranular** | | | |
| Raloxifene HCl | Active | 25.0 | 60.0 |
| Lactose monohydrate | Filler | 13.38 | 32.1 |
| Citric acid, anhydrous | Acidifying Agent | 0.13 | 0.3 |
| Polyvidon (PVP) | Binder | 5.0 | 12.0 |
| Crospovidone | Disintegrant | 6.0 | 14.4 |

| **Extragranular** | | | |
|---|---|---|---|
| Lactose anhydrous | Filler | 50.0 | 120.0 |
| Magnesium stearate | Lubricant | 0.5 | 1.2 |
| Water (removed during processing) | - | - | - |
| | **TOTAL** | 100 | 240 |

**Table B - Tablets with 13% MCC made with wet granulation**

| **Ingredient** | **Function** | **Ratio (%)** | **Unit Ratio (mg)** |
|---|---|---|---|
| **Intragranular** | | | |
| Raloxifene HCl | Active | 25.0 | 60.0 |
| Microcrystalline Cellulose | Filler | 13.3 | 31.9 |
| Citric acid, anhydrous | Acidifying Agent | 0.2 | 0.5 |
| Polyvidon (PVP) | Binder | 5.0 | 12.0 |
| Crospovidone | Disintegrant | 6.0 | 14.4 |

| **Extragranular** | | | |
|---|---|---|---|
| Lactose anhydrous | Filler | 50.0 | 120.0 |
| Magnesium stearate | Lubricant | 0.5 | 1.2 |
| Water (removed during processing) | - | - | - |
| | **TOTAL** | 100.0 | 240.0 |

**Table C -Tablets with 23% MCC made with wet granulation**

| **Ingredient** | **Function** | **Ratio (%)** | **Unit Ratio (mg)** |
|---|---|---|---|
| **Intragranular** | | | |
| Raloxifene HCl | Active | 25.0 | 60.0 |
| Microcrystalline Cellulose | Filler | 23.3 | 55.9 |
| Citric acid, anhydrous | Acidifying Agent | 0.2 | 0.5 |
| Polyvidon (PVP) | Binder | 5.0 | 12.0 |
| Crospovidone | Disintegrant | 6.0 | 14.4 |

| **Extragranular** | | | |
|---|---|---|---|
| Lactose anhydrous | Filler | 40.0 | 96.0 |
| Magnesium stearate | Lubricant | 0.5 | 1.2 |
| Water (removed during processing) | - | - | - |
| | **TOTAL** | 100.0 | 240.0 |

**Table D -Tablets with 62.5% MCC (no lactose) made with wet granulation**

| **Ingredient** | **Function** | **Ratio (%)** | **Unit Ratio (mg)** |
|---|---|---|---|
| **Intragranular** | | | |
| Raloxifene HCl | Active | 25.0 | 60.0 |
| Microcrystalline Cellulose | Filler | 12.5 | 32.4 |
| Polysorbate 80 | Surfactant | 1.0 | 2.4 |
| Polyvidon (PVP) | Binder | 5.0 | 12.0 |
| Crospovidone | Disintegrant | 6.0 | 14.4 |

| **Extragranular** | | | |
|---|---|---|---|
| Microcrystalline Cellulose | Filler | 50.0 | 120.0 |
| Magnesium stearate | Lubricant | 0.5 | 1.2 |
| Water (removed during processing) | - | - | - |

The above tablets were subjected to a dissolution test as described above. The profile for the average dissolution of each tablet is represented in Fig 1. The tablet of Table D having no lactose and approx. 62% MCC released the least amount of raloxifene while the tablet of Table A having no MCC and approx. 63% lactose performed somewhat better and achieved approximately 85% release of raloxifene in 30 minutes. But the tablets of Tables B and C having 13%MCC/50% lactose and 23%MCC/40% lactose performed the best; achieving more than 90% release in 30 minutes and more than 95% release in 60 minutes.

### Example 2

Tablets according to the below compositions were made via direct compression. Specifically, Raloxifene was added to the rest of ingredients except for magnesium stearate and mixed using a low shear blender. Magnesium stearate was added and blended with the rest of the powder for lubrication. The blend was then compressed into tablets.

### Without MCC (Direct Compression, 240mg tablet weight)

| Ingredient | Function | Ratio (%) | Unit Ratio (mg) |
|---|---|---|---|
| Raloxifene HCl | Active Ingredient | 25.0 | 60.0 |
| Colloidal Silicon dioxide | Flow Aid | 0.1 | 0.2 |
| Polyvidon (PVP) | Binder | 5.0 | 12.0 |
| Lactose monohydrate | Filler | 63.4 | 72.0 |
| Crospovidone | Disintegrant | 6.0 | 14.4 |
| Magnesium stearate | Lubricant | 0.5 | 1.2 |
| | **TOTAL** | 100.0 | 240.0 |

### With MCC (Direct Compression, 240mg tablet weight)

| Ingredient | Function | Ratio (%) | Unit Ratio (mg) |
|---|---|---|---|
| Raloxifene HCl | Active Ingredient | 25.0 | 60.0 |
| Colloidal Silicon dioxide | Flow Aid | 0.1 | 0.2 |
| Polyvidon (PVP | Binder | 5.0 | 12.0 |
| Microcrystalline Cellulose | Filler | 13.4 | 32.2 |
| Lactose monohydrate | Filler | 50.0 | 120.0 |
| Crospovidone | Disintegrant | 6.0 | 14.4 |
| Magnesium stearate | Lubricant | 0.5 | 1.2 |
| | **TOTAL** | 100.0 | 240.0 |

The above tablets were subjected to dissolution testing. The profile for the average dissolution of each tablet is represented in Fig. 2. The tablet containing both MCC and lactose release more raloxifene at 30 and 60 minutes than the tablet that contained only lactose.

### Example 3

Tablets were made by direct compression using generally the method described above in Example 2 and using different raloxifene particles sizes; i.e., non-micronized versus micronized raloxifene. The non-micronized raloxifene had a d₅₀ of 27 microns and a d₉₀ of 61 microns. The micronized raloxifene had a d₅₀ of 16 microns and a d₉₀ of 30 microns. The tablet composition for the micronized and non-micronized tablets is set forth below.

| **Ingredient** | **Function** | **Ratio (%)** | **Unit Ratio (mg)** |
|---|---|---|---|
| Raloxifene HCl | Active | 25.0 | 60.0 |
| Aerosil 200 VV Pharma | Flow Aid | 0.1 | 0.2 |
| Polyvidon (PVP) | Binder | 5.0 | 12.0 |
| Microcrystalline Cellulose | Filler | 13.4 | 32.2 |
| Crospovidone | Disintegrant | 6.0 | 14.4 |
| Lactose Anhydrous | Filler | 50.0 | 120.0 |
| Magnesium stearate | Lubricant | 0.5 | 1.2 |
| | **TOTAL** | 100.0 | 240.0 |

The tablets made with micronized or non-micronized raloxifene were subjected to a dissolution test. The profile for the average dissolution of each tablet is represented in Fig 3. Surprisingly the tablets made with non-micronized raloxifene released more raloxifene at both 30 and 60 minutes than the tablets made using micronized raloxifene.

### Example 4

Tablets having the following compositions were made via direct compression as described above. The tablet compositions were as follows:

### (Direct Compression, 240mg tablet weight)

| Ingredient | Function | Ratio (%) | Unit Ratio (mg) |
|---|---|---|---|
| Raloxifene HCl | Active Ingredient | 25.0 | 60.0 |
| Colloidal Silicon dioxide | Flow Aid | 0.1 | 0.2 |
| Polyvidon (PVP) | Binder | 5.0 | 12.0 |
| Microcrystalline Cellulose | Filler | 13.4 | 32.2 |
| Lactose monohydrate | Filler | 50.0 | 120.0 |
| Crospovidone | Disintegrant | 6.0 | 14.4 |
| Magnesium stearate | Lubricant | 0.5 | 1.2 |
| | **TOTAL** | 100.0 | 240.0 |

### (Direct Compression, 350mg tablet weight)

| Ingredient | Function | Ratio (%) | Unit Ratio (mg) |
|---|---|---|---|
| Raloxifene HCl | Active Ingredient | 17.1 | 60.0 |
| Colloidal Silicon dioxide | Flow Aid | 0.05 | 0.2 |
| Polyvidon (PVP | Binder | 5.0 | 17.5 |
| Microcrystalline Cellulose | Filler | 21.3 | 74.6 |
| Lactose monohydrate | Filler | 50.0 | 175.0 |
| Crospovidone | Disintegrant | 6.0 | 21.0 |
| Magnesium stearate | Lubricant | 0.5 | 1.8 |
| | **TOTAL** | 100.0 | 350.0 |

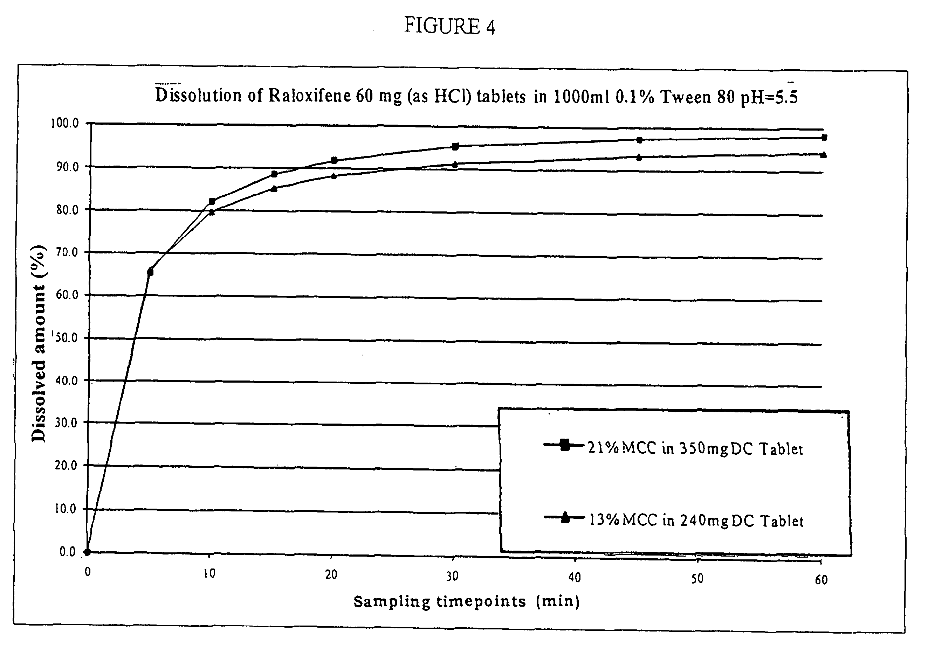

The above tablets were subjected to a dissolution test. The profile for the average dissolution of each tablet is represented in Fig. 4. Note that the larger tablet weight had superior dissolution even though the same proportions of excipients were used.

### Example 5

Tablets having the following compositions were made via wet granulation as described above. The tablet compositions were as follows:

**Table 1: Tablets with MCC and no Citric Acid (WG process)**

| **Ingredient** | **Function** | **Unit (%)** | **Unit weight (mg)** |
|---|---|---|---|
| Raloxifene HCl | Active | 25.0 | 60.0 |
| Microcrystalline Cellulose | Filler | 13.5 | 32.4 |
| Polyvidon (PVP) | Binder | 5.0 | 12.0 |
| Crospovidone | Disintegrant | 6.0 | 14.4 |
| Lactose anhydrous | Filler | 50.0 | 120.0 |
| Magnesium stearate | Lubricant | 0.5 | 1.2 |
| Water (removed during processing) | - | - | - |
| | **TOTAL** | 100.0 | 240.0 |

**Table 2: Tablets with MCC and Citric Acid (WG process)**

| **Ingredient** | **Function** | **Unit (%)** | **Unit weight (mg)** |
|---|---|---|---|
| Raloxifene HCl | Active | 25.0 | 60.0 |
| Microcrystalline Cellulose | Filler | 13.3 | 31.9 |
| Citric acid, anhydrous | Acidifying Agent | 0.2 | 0.5 |
| Polyvidon (PVP) | Binder | 5.0 | 12.0 |
| Crospovidone | Disintegrant | 6.0 | 14.4 |
| Lactose anhydrous | Filler | 50.0 | 120.0 |
| Magnesium stearate | Lubricant | 0.5 | 1.2 |
| Water (removed during processing) | - | - | - |
| | **TOTAL** | 100.0 | 240.0 |

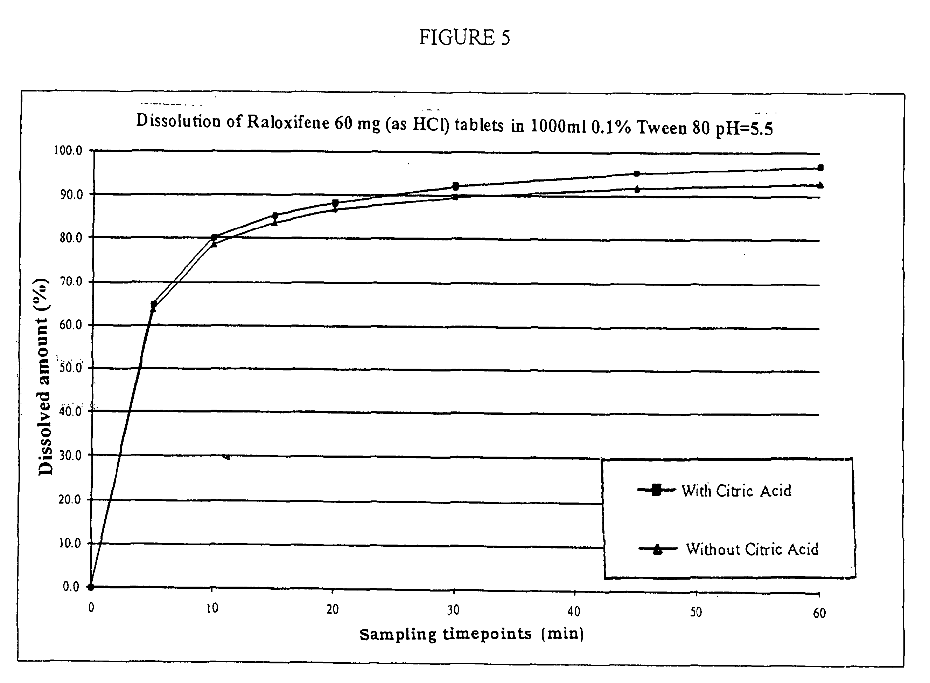

The above tablets were subjected to a dissolution test. The profile for the average dissolution of each tablet is represented in Fig. 5. The tablets that contained 0.2% citric acid released more raloxifene at 30 and 60 minutes than the tablets that did not contain citric acid.

### Example 6

Tablets having the following compositions were made via wet granulation as described above. The tablet compositions were as follows:

**Tablet 6A -no citric acid**

| **N°** | **FORMULA** | **LOT No:** | **UNIT WEIGHT(%)** | **UNIT WEIGHT (mg)** | **LOT WEIGHT (g)** |
|---|---|---|---|---|---|
| 1 | RXF.HCl (non-micronised) | 0808053 | 25.0 | 60.0 | 37.5 |
| 2 | Lactose monohydrate | II.lac.021.01H2 | 13.5 | 32.4 | 20.25 |
| 3 | Polyvidon K 30 | II.pvp.004.01G1 | 5.0 | 12.0 | 7.5 |
| 4 | Crospovidone | II.cpv.002.02I1 | 6.0 | 14.4 | 9.0 |
| 5 | Lactose anhydrous | II.lac.005.01I1 | 50.0 | 120.0 | 75.0 |
| 6 | Magnesium stearate | II.mgs.003.01F1 | 0.5 | 1.2 | 0.75 |
| 7 | Water (removed during processing) | - | - | - | 13 |
| **TOTAL** | | | 100.0 | 240.0 | 150.0 |

**Tablet 6B with citric acid**

| **N°** | **FORMULA** | **LOT No:** | **UNIT WEIGHT(%)** | **UNIT WEIGHT (mg)** | **LOT WEIGHT (g)** |
|---|---|---|---|---|---|
| 1 | RXF.HCl (non-micronised) | 0808053 | 25.0 | 60.0 | 37.5 |
| 2 | Lactose monohydrate | II.lac.021.01H2 | 13.375 | 32.1 | 20.06 |
| 3 | Citric acid, anhydrous | II.cit.006.01I5 | 0.125 | 0.3 | 0.19 |
| 4 | Polyvidon K 30 | II.pvp.004.01F1 | 5.0 | 12.0 | 7.5 |
| 5 | Crospovidone | II.cpv.002.02I1 | 6.0 | 14.4 | 9.0 |
| 6 | Lactose anhydrous | II.lac.005.01I1 | 50.0 | 120.0 | 75.0 |
| 7 | Magnesium stearate | II.mgs.003.01F1 | 0.5 | 1.2 | 0.75 |
| 8 | Water (removed during processing) | - | - | - | 11 |
| **TOTAL** | | | 100.0 | 240.0 | 150.0 |

The tablets 6B displayed faster and more complete release of raloxifene than tablets 6A. For example, at five minutes tablets 6B had released over 55% of the raloxifene while the 6A tablets had released about 45%. Though the 6A tablets close this gap, the 6B tablets always have released more raloxifene than the 6A tablets higher, typically by 3-5%.

The invention having been described it will be obvious that the same may be varied in many ways and all such modifications are contemplated as being within the scope of the invention as defined by the following claims.

## Claims

1. A pharmaceutical tablet, comprising:
(a) an effective amount of non-micronized raloxifene, or a pharmaceutically acceptable salt thereof, with particle size having a d₉₀ equal or larger than 50 microns and/or a d₅₀ equal or larger than 25 microns;
(b) at least 25% by weight of lactose, preferably 40 to 60% by weight of said tablet;
(c) 5 to 25% by weight of microcrystalline cellulose, preferably 10 to 20% by weight of said tablet;
(d) optionally a binder; and
(e) optionally a disintegrant.

2. The tablet according to claim 1, which contains 1 to 10% by weight of said binder, and preferably said binder is a polyvinylpyrrolidone.

3. The tablet according to claim 1 or 2, which contains 1-10% by weight of said disintegrant, and preferably said disintegrant is a crospovidone.

4. The tablet according to any one of claims 1 to 3, which further comprises 0.1 to 5.0% by weight of an organic acid.

5. The tablet according to claim 4, wherein said organic acid is citric acid, and preferably said citric acid is contained in an amount of 0.1-1.0% by weight.

6. The tablet according to any one of claims 1 to 5, wherein the weight ratio of microcrystalline cellulose to lactose is within the range of 1:1-12 (parts : parts), preferably 1:1.5-10, more preferably 1:2-5, respectively.

7. The tablet according to any one of claims 1 to 6, wherein said raloxifene is raloxifene hydrochloride.

8. The tablet according to any one of claims 1 to 7, wherein said raloxifene has a d₉₀ of 51 to 75 microns.

9. The tablet according to any one of claims 1 to 8, wherein said raloxifene has a d₅₀ of 26 to 40 microns.

10. The tablet according to any one of claims 1 to 9, wherein said raloxifene comprises 10 to 30% by weight of said tablet.

11. The tablet according to any one of claims 1 to 10, wherein said tablet exhibits at least 85% raloxifene release in 30 minutes using USP apparatus Type II, in 1 liter aqueous solution containing 0.1% polysorbate 80 at paddle speed of 50 rpm.

12. A process for making a tablet, which comprises:
(1) mixing non-micronized raloxifene, or a pharmaceutically acceptable salt thereof, having an average particle size of at least 25 microns and/or a d₉₀ of at least 50 microns; microcrystalline cellulose; a disintegrant; and optionally a binder to form a blend;
(2) wet granulating said blend with water to form granules;
(3) mixing said granules with at least lactose to form a tablet blend; and
(4) tabletting said tablet blend by compression to form at least one tablet;
wherein lactose comprises at least 25% by weight, and preferably 40 to 60% by weight of said tablet and said microcrystalline cellulose comprises 5 to 25% by weight of said tablet.

13. The process according to claim 12, wherein said water used in said granulating step contains an organic acid.

14. A process for making a tablet without the aid of water, which comprises:
(1) blending in one or more steps at least non-micronized raloxifene, or a pharmaceutically acceptable salt thereof, having an average particle size of at least 25 microns and/or a d₉₀ of at least 50 microns; microcrystalline cellulose; lactose; a binder; and a disintegrant to form a tablet blend; and
(2) tabletting said tablet blend by compression to form at least one tablet;
wherein lactose comprises at least 25% by weight, and preferably 40 to 60% by weight of said tablet and said microcrystalline cellulose comprises 5 to 25% by weight of said tablet.

## Patentansprüche

1. Pharmazeutische Tablette, umfassend:
(a) eine wirksame Menge nicht-mikronisiertes Raloxifen oder ein pharmazeutisch verträgliches Salz davon, mit einer Partikelgröße mit einer d₉₀ gleich odergrößer als 50 Mikrometer und/oder einer d₅₀ gleich oder größer als 25 Mikrometer;
(b) mindestens 25 Gew.-% Lactose, vorzugsweise 40 bis 60 Gew.-% der Tablette;
(c) 5 bis 25 Gew.-% mikrokristalline Cellulose, vorzugsweise 10 bis 20 Gew.-% der Tablette;
(d) gegebenenfalls ein Bindemittel; und
(e) gegebenenfalls ein Sprengmittel.

2. Tablette nach Anspruch 1, die 1 bis 10 Gew.-% des Bindemittels enthält und wobei das Bindemittel vorzugsweise ein Polyvinylpyrrolidon ist.

3. Tablette nach Anspruch 1 oder 2, die 1-10 Gew.-% des Sprengmittels enthält und wobei das Sprengmittel vorzugsweise ein Crospovidon ist.

4. Tablette nach einem der Ansprüche 1 bis 3, die des Weiteren 0,1 bis 5,0 Gew-% einer organischen Säure umfasst.

5. Tablette nach Anspruch 4, wobei die organische Säure Zitronensäure ist und die Zitronensäure vorzugsweise in einer Menge von 0,1 bis 1,0 Gew.-% enthalten ist.

6. Tablette nach einem der Ansprüche 1 bis 5, wobei das Gewichtsverhältnis von mikrokristalliner Cellulose zu Lactose innerhalb des Bereichs von 1:1-12 (Anteile : Anteile), vorzugsweise 1:1,5-10 bzw. stärker vorzugsweise 1:2-5 beträgt.

7. Tablette nach einem der Ansprüche 1 bis 6, wobei das Raloxifen Raloxifenhydrochlorid ist.

8. Tablette nach einem der Ansprüche 1 bis 7, wobei das Raloxifen eine d₉₀ von 51 bis 75 Mikrometer aufweist.

9. Tablette nach einem der Ansprüche 1 bis 8, wobei das Raloxifen eine d₅₀ von 26 bis 40 Mikrometer aufweist.

10. Tablette nach einem der Ansprüche 1 bis 9, wobei das Raloxifen 10 bis 30 Gew.-% der Tablette umfasst.

11. Tablette nach einem der Ansprüche 1 bis 10, wobei die Tablette eine mindestens 85 %ige Raloxifen-Freisetzung in 30 Minuten unter Verwenden eines USP-Apparats Typ II, in 1 Liter wässriger Lösung, enthaltend 0,1 % Polysorbat 80, bei einer Messflügeldrehzahl von 50 UpM, zeigt.

12. Verfahren zum Herstellen einer Tablette, das umfasst:
(1) Mischen von nicht-mikronisiertem Raloxifen oder einem pharmazeutisch verträglichen Salz davon, mit einer durchschnittlichen Partikelgröße von mindestens 25 Mikrometer und/oder einer d₉₀ von mindestens 50 Mikrometer; mikrokristalliner Cellulose; einem Sprengmittel; und gegebenenfalls einem Bindemittel, um eine Mischung zu bilden;
(2) Feuchtgranulieren der Mischung mit Wasser, um Granulat zu bilden;
(3) Mischen des Granulats mit mindestens Lactose, um eine Tablettenmischung herzustellen; und
(4) Tablettieren der Tablettenmischung durch Kompression, um mindestens eine Tablette zu bilden;
wobei Lactose mindestens 25 Gew.-% und vorzugsweise 40 bis 60 Gew.-% der Tablette umfasst und die mikrokristalline Cellulose 5 bis 25 Gew.-% der Tablette umfasst.

13. Verfahren nach Anspruch 12, wobei das in dem Granulierschritt verwendete Wasser eine organische Säure enthält.

14. Verfahren zum Herstellen einer Tablette ohne die Hilfe von Wasser, das umfasst:
(1) Mischen in einem oder mehreren Schritten von mindestens nichtmikronisiertem Raloxifen oder einem pharmazeutisch verträglichen Salz davon, mit einer durchschnittlichen Partikelgröße von mindestens 25 Mikrometer und/oder einer d₉₀ von mindestens 50 Mikrometer; mikrokristalliner Cellulose; Lactose, einem Bindemittel; und einem Sprengmittel, um eine Tablettenmischung herzustellen; und
(2) Tablettieren der Tablettenmischung durch Kompression, um mindestens eine Tablette zu bilden;
wobei Lactose mindestens 25 Gew.-% und vorzugsweise 40 bis 60 Gew.-% der Tablette umfasst und die mikrokristalline Cellulose 5 bis 25 Gew.-% der Tablette umfasst.

## Revendications

1. Un comprimé pharmaceutique comprenant:
(a) une quantité efficace de raloxifène non micronisé, ou d'un sel pharmaceutiquement acceptable en dérivant, dont la dimension des particules présente une d₉₀ égale ou supérieure à 50 microns et/ou une d₅₀ égale ou supérieure à 25 microns;
(b) au moins 25% en poids de lactose, de préférence 40 à 60% en poids dudit comprimé;
(c) de 5 à 25% en poids de cellulose microcristalline, de préférence de 10 à 20% en poids dudit comprimé;
(d) éventuellement un liant; et
(e) éventuellement un délitant.

2. Le comprimé selon la revendication 1, qui contient 1 à 10% en poids dudit liant, et de préférence ledit liant est une polyvinylpyrrolidone.

3. Le comprimé selon la revendication 1 ou 2, qui contient 1-10% en poids dudit délitant, et de préférence ledit délitant est une crospovidone.

4. Le comprimé selon une quelconque des revendications 1 à 3, qui comprend en outre 0,1 à 5,0% en poids d'un acide organique.

5. Le comprimé selon la revendication 4, dans lequel ledit acide organique est l'acide citrique, et de préférence ledit acide citrique est contenu en une proportion de 0,1-1,0% en poids.

6. Le comprimé selon une quelconque des revendications 1 à 5, dans lequel le ratio massique cellulose microcristalline:lactose est dans la gamme de 1:1-12 (parties/parties), de préférence 1:1,5-10, mieux encore 1:2-5, respectivement.

7. Le comprimé selon une quelconque des revendications 1 à 6, dans lequel ledit raloxifène est du chlorhydrate de raloxifène.

8. Le comprimé selon une quelconque des revendications 1 à 7, dans lequel ledit raloxifène présente une d₉₀ de 51 à 75 microns.

9. Le comprimé selon une quelconque des revendications 1 à 8, dans lequel ledit raloxifène présente une d₅₀ de 26 à 40 microns.

10. Le comprimé selon une quelconque des revendications 1 à 9, dans lequel ledit raloxifène représente de 10 à 30% en poids dudit comprimé.

11. Le comprimé selon une quelconque des revendications 1 à 10, dans lequel ledit comprimé donne lieu à la libération d'au moins 85% de raloxifène en 30 minutes en utilisant un appareil USP de type II, dans 1 litre de solution aqueuse contenant 0,1% de polysorbate 80 à une vitesse d'agitation de 50 tours par minute.

12. Un procédé de fabrication d'un comprimé, qui comprend:
(1) le mélange de raloxifène non micronisée, ou d'un sel pharmaceutiquement acceptable en dérivant, présentant une taille moyenne de particules d'au moins 25 microns et/ou une d₉₀ d'au moins 50 microns, de cellulose microcristalline, d'un délitant, et éventuellement d'un liant, pour former un mélange;
(2) la granulation par voie humide dudit mélange avec de l'eau pour former des granules;
(3) le mélange desdits granules avec au moins du lactose pour former un mélange pour comprimé; et
(4) le pastillage dudit mélange pour comprimé par compression pour former au moins un comprimé;
dans lequel ledit lactose représente au moins 25% en poids, et de préférence 40 à 60% en poids du dudit comprimé et ladite cellulose microcristalline représente 5 à 25% en poids dudit comprimé.

13. Le procédé selon la revendication 12, dans lequel ladite eau utilisée dans ladite étape de granulation contient un acide organique.

14. Un procédé de fabrication d'un comprimé sans l'aide d'eau, qui comprend:
(1) le mélange en une ou plusieurs étapes d'au moins du raloxifène non micronisé, ou d'un sel pharmaceutiquement acceptable en dérivant, présentant une taille moyenne de particules d'au moins 25 microns et/ou une d₉₀ d'au moins 50 microns, de cellulose microcristalline, de lactose, d'un liant et d'un délitant pour former un mélange pour comprimé; et
(2) le pastillage dudit mélange pour comprimé par compression pour former au moins un comprimé;
dans lequel ledit lactose représente au moins 25% en poids, et de préférence 40 à 60% en poids dudit comprimé et ladite cellulose microcristalline représente 5 à 25% en poids dudit comprimé.
